(19) Europäisches Patentamt
European Patent Office
Office européen des brevets

(11) Numéro de publication: **0 275 760 B1**

(12) **FASCICULE DE BREVET EUROPEEN**

(45) Date de publication de fascicule du brevet: **01.04.92**

(51) Int. Cl.[5]: **C07D 211/42**, C07D 223/08, A61K 31/445, A61K 31/55

(21) Numéro de dépôt: **87402886.3**

(22) Date de dépôt: **17.12.87**

# (54) Pipéridines, ou azépines substituées, leur préparation et leur application en thérapeutique.

(30) Priorité: **23.12.86 FR 8618082**

(43) Date de publication de la demande:
**27.07.88 Bulletin 88/30**

(45) Mention de la délivrance du brevet:
**01.04.92 Bulletin 92/14**

(84) Etats contractants désignés:
**AT BE CH DE ES FR GB GR IT LI NL SE**

(56) Documents cités:
**FR-A- 2 000 160**
**FR-M- 8 431**

(73) Titulaire: **RIOM LABORATOIRES- C.E.R.M. "R.L.-CERM" - (S.A.)**
**Route de Marsat B.P. 140**
**F-63203 Riom Cédex(FR)**

(72) Inventeur: **Carlier, Patrick**
**La Gravière 10, Fonfreyde**
**F-63140 Chatel-Guyon(FR)**
Inventeur: **Simond, Jacques Aimé Louis**
**Rue des Thuilets, Mirefleurs**
**F-63730 Les-Martres-de-Veyre(FR)**
Inventeur: **Monteil, André Jean-Claude**
**Route de Prompsat, Les Grosliers**
**F-63140 Chatel-Guyon(FR)**

(74) Mandataire: **Hermans, Franciscus G.M. et al**
**Patent Department AKZO N.V. Pharma Division P.O. Box 20**
**NL-5340 BH Oss(NL)**

**EP 0 275 760 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouvelles pipéridines ou azépines substituées, leur préparation et leur application en thérapeutique.

Plus précisément, les 1-alkyl-2-alkoxyméthyl-3-alkynyloxy pipéridines ou azépines de l'invention répondent à la formule générale suivante :

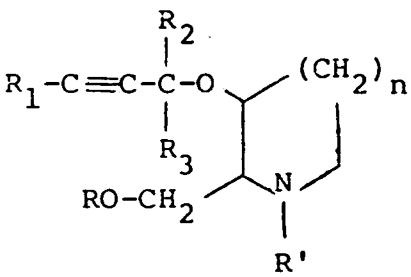

dans laquelle R et R' représentent un radical alkyle ayant 1 à 7 atomes de carbone ; $R_1$ représente l'hydrogène ou un radical alkyle ayant 1 à 7 atomes de carbone ; $R_2$ et $R_3$ représentent séparément un radical alkyle ayant 1 à 7 atomes de carbone ou le radical phényle ou ensemble avec l'atome de carbone auquel ils sont liés un radical cycloalkyle ayant au plus 7 atomes de carbone ; n pouvant prendre les valeurs 2 ou 3.

L'invention concerne aussi les sels desdits composés avec les acides organiques ou inorganiques pharmaceutiquement acceptables, tels que les acides chlorhydrique, fumarique, maléique, citrique ou succinique, ces acides n'étant mentionnés qu'à titre illustratif mais sans constituer une limitation.

Selon une réalisation préférée, les substituants R', $R_1$, $R_2$ ou $R_3$ représentent le radical méthyle.

Lorsque $R_2$ et $R_3$ représentent ensemble un radical cycloalkyle, une réalisation préférée est constituée par le radical cyclohexyle.

Dans la définition du substituant R, les radicaux étyle et isobutyle constituent les réalisations préférées.

Les composés de l'invention comportant des atomes de carbone asymétriques, le racémique et/ou les isomères optiques séparés ainsi qu'un mélange de ceux-ci font également partie de l'invention.

Les études pharmacologiques ont montré que les composés de l'invention possédaient d'intéressantes propriétés permettant leur application en thérapeutique humaine dans le traitement des troubles cardiovasculaires.

Les composés de l'invention peuvent être préparés à partir du 2-(α-hydroxy-β-alkoxy) éthyl pyrrolidine ou pipéridine, selon le schéma réactionnel ci-après :

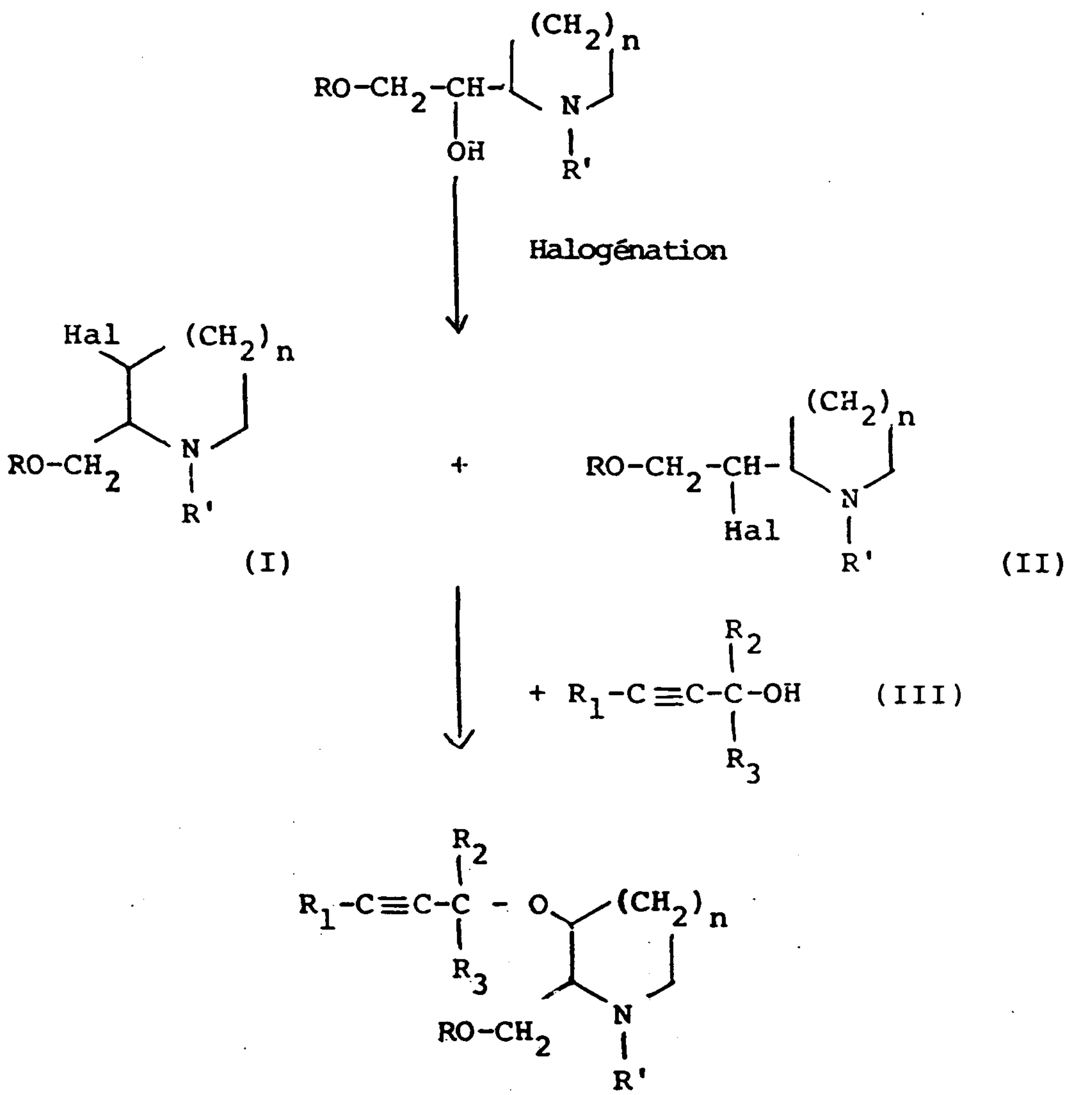


Dans une première étape, on effectue l'halogénation d'une 2-(α-hydroxy-β-alkoxy) éthyl pyrrolidine ou pipéridine au moyen d'un agent habituel d'halogénation tel que $SOCl_2$, $PBr_3$ dans un solvant tel que le chloroforme, à une température comprise entre la température ambiante et la température de reflux de solvant.

Le dérivé halogéné formé peut avoir la structure I ou la structure II, ou bien être un mélange des deux types de structure.

Dans la première étape, on obtient de façon prépondérante le composé de type I (correspondant à un agrandissement de cycle azoté) lorsque n = 2, alors qu'on obtient seulement des composés de type II lorsque n = 3, l'agrandissement du cycle s'effectuant dans la deuxième étape.

Dans la deuxième étape, le produit obtenu à l'étape précédente est mis à réagir avec un alcool acétylénique de formule III, de préférence par transfert de phase en présence d'une base forte telle que l'hydroxyde de sodium à 50 - 70 % et d'un catalyseur tel que le chlorure de benzyle triéthylammonium, en ajoutant, si la viscosité du milieu l'exige, un solvant tel que le toluène ou le chlorure de méthylène.

A l'issue de cette étape, les composés de l'invention sont extraits du mélange réactionnel par les méthodes habituelles (par exemple extraction à l'éther ou au chlorure de méthylène) puis purifiés par chromatographie liquide préparative.

Les exemples ci-après illustrent plus en détails la préparation des composés de l'invention.

EXEMPLE 1 : 1-méthyl-2-[(2-méthylpropoxy) méthyl]-3-[[1-(1-proponyl) cyclohexyl]oxy]pipéridine

Dans une première étape, on a introduit 100 g (0,5 M) de 1-méthyl-2-(α-hydroxy-β-isobutoxy) éthyl pyrrolidine dans un réacteur contenant 1 l de chloroforme anhydre, puis chauffé à 50 °C et introduit goutte

à goutte une solution de 82 ml de chlorure de thionyle dans 80 ml de chloroforme anhydre, et maintenu 4 heures en chauffant à reflux de solvant. On a ensuite évaporé le solvant et repris le résidu par 1 l d'acide chlorhydrique à 3 %, puis lavé au chlorure de méthylène, alcalinisé à la lessive de soude puis extrait au chlorure de méthylène. Après séchage sur sulfate de sodium et évaporation du solvant, on a distillé le résidu et obtenu 64 g de 1-méthyl 2-isobutoxyméthyl-3-chloro-pipéridine ayant pour point d'ébullition

Eb (66,65 Pa)($Eb_{0,5}$) = 92 - 93 ° C

Dans une deuxième étape, on a introduit dans un réacteur 22 g d'hydroxyde de sodium, 110 ml de toluène, 2,2 g de chlorure de benzyltriéthylammonium, 22 ml d'eau et 21 g de 1-(1-propynyl) cyclohexanol, puis on a ajouté goutte à goutte une solution de 22 g du dérivé chloré précédemment obtenu dans 60 ml de toluène et chauffé le mélange à 70 - 80 ° C pendant 10 heures. Après refroidissement, on a décanté, lavé la phase aqueuse au chlorure de méthylène, rassemblé les phases organiques qu'on a lavées à l'eau et séchées sur sulfate de sodium.

Après évaporation et distillation, on a obtenu 13,5 g de produit brut ayant pour point d'ébullition

Eb (66,65 Pa)($Eb_{0,5}$) = 132 - 135 ° C

On a ensuite purifié le produit obtenu par chromatographie liquide préparative sur gel de silice en utilisant un solvant ayant la composition suivante $CH_2Cl_2$ :

89,9 % - $CH_3OH$ : 10 % - $NH_4OH$ : 0,1 %.

On a ainsi obtenu 6 g de composé du titre ayant pour indice $n_D^{20}$ = 1,4850 et pour analyse élémentaire :

| | C % | H % | N % |
|---|---|---|---|
| Théorie | 74,72 | 10,97 | 4,36 |
| Trouvé | 73,44 | 11,04 | 4,36 |

EXEMPLE 2 : 1-méthyl-2-[(2-méthylpropoxy) méthyl]-3-[(1-méthyl-1-phényl-2-propynyl) oxy]azépine

Selon la même méthode que celle décrite dans l'exemple 1, en partant de 16 g de 1-méthyl-2-(α-hydroxy-β-isobutoxy) éthyl pipéridine en solution dans 160 ml de chloroforme anhydre et de 16 ml de chlorure de thionyle dans 16 ml de chloroforme anhydre, on a obtenu après 18 heures de chauffage à reflux 9,7 g de 1-méthyl-2-(α-chloro-β-isobutoxy) éthyl pipéridine qu'on a utilisé à l'état brut au stade suivant.

Dans la deuxième étape, en utilisant pour le transfert de phase une composition semblable à celle décrite dans l'exemple 1, on a obtenu à partir du composé du stade précédent et de 10,3 g de 3-phényl-1-butyn-3-ol, 6 g de composé du titre, à l'état brut, ayant pour point d'ébullition

Eb (26,66 Pa) ($Eb_{0,2}$) = 160 - 165 ° C

Pour purifier le produit obtenu, on a effectué une chromatographie liquide préparative sur gel de silice en utilisant comme solvant un mélange de dichlorométhane contenant des quantités croissantes de méthanol (de 0 à 4 %).

On a ainsi obtenu 2,9 g de composé purifié ayant pour indice $n_D^{20}$ = 1,5075 et pour analyse élémentaire :

| | C % | H % | N % |
|---|---|---|---|
| Théorie | 76,92 | 9,68 | 4,07 |
| Trouvé | 76,27 | 9,79 | 4,09 |

EXEMPLE 3 : 1-méthyl-2-[(1-éthoxy) méthyl]-3-[(1-méthyl-1-phényl-2-propynyl) oxy]pipéridine

En procédant comme indiqué dans l'exemple 1, dans une première étape, on a obtenu par action du chlorure de thionyle la 1-méthyl-2-éthoxy méthylène 3-chloropipéridine ayant pour point d'ébullition

Eb (1599,6 Pa) ($Eb_{12}$) = 100 °C

En mettant à réagir ce dérivé chloré avec le 3-phényl-1-butyn-3-ol pendant 5 heures en présence du mélange de transfert de phase, puis en effectuant une purification par chromatographie préparative sur gel de silice, avec le même solvant que pour l'exemple 1, on a obtenu le composé du titre ayant pour indice $n_D^{20}$ = 1,5150 et pour analyse élémentaire :

| | C % | H % | N % |
|---|---|---|---|
| Théorie | 75,71 | 9,03 | 4,65 |
| Trouvé | 75,33 | 9,02 | 4,63 |

De la même façon, on a préparé un certain nombre de composés selon l'invention, dont les caractéristiques sont résumées dans le tableau I ci-après :

TABLEAU I

| COMPOSE N° | $R_1$ | $R_2$ | $R_3$ | R | n | $n_D^{20}$ | $Eb_{mmHg}$ °C |
|---|---|---|---|---|---|---|---|
| 1 (Exemple 1) | $-CH_3$ | $-(CH_2)_5-$ | | $CH_3$<br>$CH-CH_2-$<br>$CH_3$ | 2 | 1,4852 | $E_{0,5}$ = 132-135 |
| 2 (Exemple 3) | H | 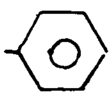 | $-CH_3$ | $-C_2H_5$ | 2 | 1,5150 | $E_{0,1}$ = 134-136 |
| 3 | H | 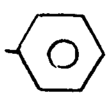 | $-CH_3$ | $CH_3$<br>$CH-CH_2-$<br>$CH_3$ | 2 | 1,5090 | $E_1$ = 93 |
| 4 (Exemple 2) | H | 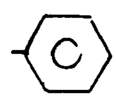 | $-CH_3$ | $CH_3$<br>$CH-CH_2-$<br>$CH_3$ | 3 | 1,5075 | $E_{0,2}$ = 160-165 |
| 5 | $-CH_3$ | $-(CH_2)_5-$ | | $CH_3$<br>$CH_3CH-CH_2-$<br>$CH_3$ | 3 | 1,4860 | $E_{0,1}$ = 130-138 |

Dans le tableau I ci-dessus, le substituant R' désigne le radical méthyle.

Les composés de l'invention se sont révélés posséder d'intéressantes propriétés anti-angineuses, avec des effets bradycardisants, antitachycardisants et coronarodilatateurs.

L'activité anticalcique a été recherchée selon la technique de Van Rossum (Arch. Int. Pharmacodyn. Ther. 143, 299-330, 1963). Pour évaluer l'activité anticalcique au niveau cardiaque, on a utilisé le muscle papillaire de lapin stimulé électriquement (fréquence 1,5 Hz ; 5 ms d'impulsion de 15 v.). Pour l'activité au niveau vasculaire, on a utilisé l'aorte de lapin découpée en spirale et maintenue dans une solution

dépourvue de $Ca^{++}$ et enrichie de $K^{+}$ (6 mg/l de KCl). Les mesures ont été effectuées 15 minutes après avoir ajouté les composés à la solution. Les paramètres classiques de pharmacologie moléculaire sont rapportés dans le tableau II.

La recherche de l'activité anti-angineuse a été évaluée en recherchant les effets hémodynamiques chez le chien anesthésié. L'animal est anesthésié au chloralose (100 $mg.kg^{-1}$ I.V.) et les paramètres suivants sont enregistrés :

. fréquence cardiaque à l'aide d'électrodes à ECG sous-cutanées reliées à un cardiotachymètre,
. le débit artériel coronaire à l'aide d'un débitmètre électromagnétique,
. la pression artérielle à l'aide d'un cathéter Mikro-tip au niveau de la crosse aortique,
. l'inotropisme évalué par la dP/dt maximale gauche par dérivation par rapport au temps de la pression intraventriculaire gauche enregistrée à l'aide d'un cathéter Mikro-tip dans le ventricule gauche,
. l'action anti-tachycardisante (inhibition des effets chronotropes positifs de l'isoprénaline).

Ces paramètres sont enregistrés en continu sur un dynographe BECKMAN. Les composés sont administrés par voie I.V. à la dose de 5 $mg.kg^{-1}$.

Les résultats sont exprimés en pourcentage de variation, la durée d'action étant indiquée entre parenthèses, en minutes (Tableau III).

TABLEAU II

| COMPOSE N° | ACTIVITE ANTICALCIQUE | |
|---|---|---|
| | CARDIAQUE | VASCULAIRE |
| 1 | 4,8 | 5,6 |
| 2 | 4,5 | 4,9 |
| 3 | 4,6 | 5,6 |
| 4 | 5,4 | 5,6 |
| 5 | 4,7 | 4,9 |

TABLEAU III

| COMPOSE N° | ACTIVITE HEMODYNAMIQUE | | | | |
|---|---|---|---|---|---|
| | FREQUENCE CARDIAQUE | DEBIT CORONAIRE | HYPOTENSION | INOTROPISME | ANTI-TACHYCARDIE |
| 1 | - 17(> 40) | + 124(10) | - 32 (5) | - 13 (3) | 0 |
| 2 | - 12(> 40) | + 114 (4) | - 9 (2) | - 19(> 39) | - 27 (17) |
| 3 | - 24(> 40) | + 44 (4) | - 55 (7) | - 61(> 40) | - 30 (17) |
| 4 | - 30(> 40) | + 64 (2) | - 50 (5) | - 32(> 40) | - 15 (17) |
| 5 | - 41(> 40) | + 25 (2) | - 58 (4) | - 50 (23) | 0 |

Ces résultats montrent que les composés de l'invention sont des anticalciques aussi bien sur le muscle cardiaque que sur le muscle vasculaire, les composés $n^{os}$ 1 et 3 étant ceux qui présentent la plus forte activité.

En ce qui concerne les effets hémodynamiques, tous ces composés peuvent être, à des degrés différents, des anti-angineux et/ou des anti-ischémiques en raison de leurs activités bradycardisante, coronarodilatatrice et anti-tachycardisante. Leur action sur la pression artérielle permet également d'envisager leur application comme anti-hypertenseur. Le composé n° 1 est celui qui présente les paramètres les plus favorables.

La toxicité a été observée par voie orale chez la souris et on n'a observé aucun décès jusqu'à 500 $mg.kg^{-1}$.

Cet ensemble de propriétés pharmacologiques permet l'application des composés de l'invention en thérapeutique humaine en tant que médicament pour le traitement des troubles cardiovasculaires, tel que l'angine de poitrine, l'ischémie ou l'hypertension.

Les composés de l'invention peuvent être administrés par voie orale ou parentérale à des doses

journalières comprises entre 0,5 mg et 10 mg par kg de poids corporel selon la méthode d'administration.

En thérapeutique humaine, on utilise de préférence une dose journalière comprise entre 50 et 500 mg.

Associés aux excipients pharmaceutiques habituels, les composés de l'invention, ou leurs sels, peuvent être mis sous forme unitaires tels que pilules, comprimés, comprimés enrobés, ou bien conditionnés en capsules. En utilisant des liquides appropriés, les composés de l'invention peuvent aussi être utilisés en préparation injectable, ou en préparation orale sous forme de solutions, suspensions ou émulsions.

**Revendications**

**Revendications pour les Etats contractants suivants : AT, BE, CH, DE, FR, GB, IT, LI, NL, SE**

1. Composés de formule :

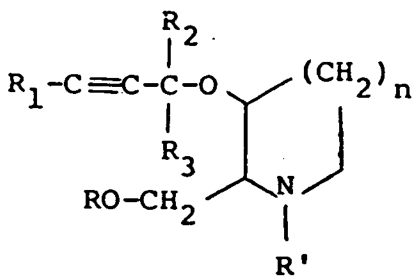

dans laquelle R et R′ représentent un radical alkyle ayant 1 à 7 atomes de carbone ; $R_1$ représente l'hydrogène ou un radical alkyle ayant 1 à 7 atomes de carbone ; $R_2$ et $R_3$ représentent séparément un radical alkyle ayant 1 à 7 atomes de carbone ou le radical phényle ou ensemble avec l'atome de carbone auquel ils sont liés un radical cycloalkyle ayant au plus 7 atomes de carbone ; n pouvant prendre les valeurs 2 ou 3, et leurs sels pharmaceutiquement acceptables.

2. Composés selon la revendication 1, caractérisés en ce que les substituants R′, $R_1$, $R_2$ ou $R_3$ représentent le radical méthyle.

3. Composés selon la revendication 1, caractérisés en ce que $R_2$ et $R_3$ représentent ensemble le radical cyclohexyle.

4. Composés selon la revendication 1, caractérisés en ce que R représente le radical éthyle ou le radical isobutyle.

5. Procédé pour l'obtention des composés selon l'une quelconque des revendications 1 à 4, caractérisé en ce qu'on fait agir un dérivé halogéné de formule I ou II

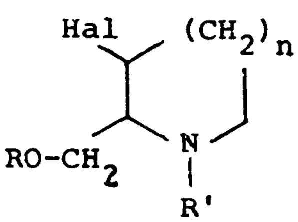

(I)

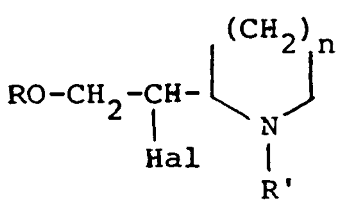

(II)

(dans lesquelles R et R′ ont les significations de la revendication 1 et " Hal " désigne un halogène) avec un alcool acétylénique de formule III

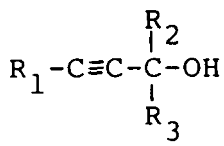

(III)

(dans laquelle $R_1$, $R_2$ et $R_3$ ont les significations de la revendication 1)
éventuellement suivi par la transformation en un sel pharmaceutiquement acceptable.

6. Procédé selon la revendication 5, caractarisé en ce que la réaction est effectuée par transfert de phase, en présence d'une base forte et d'un catalyseur tel que le chlorure de benzyltriéthylammonium.

7. Procédé selon la revendication 5, caractérisé en ce qu'on obtient les dérivés halogénés de formule I ou II en traitant une 2-(α-hydroxy-β-alkoxy) éthyl pyrrolidine ou-pipéridine par un agent d'halogénation.

8. Procédé selon la revendication 7, caractérisé en ce que l'halogénation est effectuée au moyen de chlorure de thionyl.

9. Composition pharmaceutique utile en tant que médicament, caractérisée en ce qu'elle contient à titre de principe actif au moins un des composés selon les revendications 1 à 4.

**Revendications pour les Etats contractants suivants : ES, GR**

1. Procédé pour l'obtention des composés de formule:

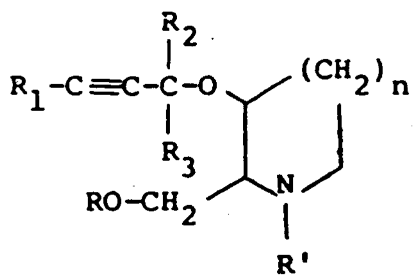

dans laquelle R et R' représentent un radical alkyle ayant 1 à 7 atomes de carbone; $R_1$ représente l'hydrogène ou un radical alkyle ayant 1 à 7 atomes de carbone; $R_2$ et $R_3$ représentent séparément un radical alkyle ayant 1 à 7 atomes de carbone ou le radical phényle ou ensemble avec l'atome de carbone auquel ils sont liés un radical cycloalkyle ayant au plus 7 atomes de carbone; n pouvant prendre les valeurs 2 ou 3, et leurs sels pharmaceutiquement acceptables, caractérisé en ce qu'on fait agir un dérivé halogéné de formule I ou II

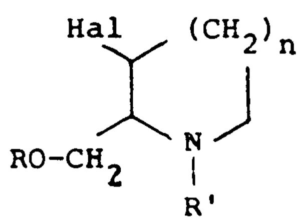

(I)

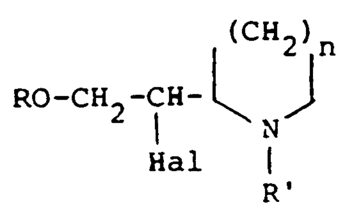

(II)

(dans lesquelles R et R' ont les significations données ci-dessus et "Hal" désigne un halogène) avec un alcool acétylénique de formule III

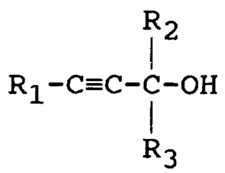

(III)

(dans laquelle $R_1$, $R_2$ et $R_3$ ont les significations données ci-dessus) éventuellement suivi par la transformation en un sel pharmaceutiquement acceptable.

**2.** Procédé selon la revendication 1, caractérisés en ce que les substituants R', $R_1$, $R_2$ ou $R_3$ représentent le radical méthyle.

**3.** Procédé selon la revendication 1, caractérisés en ce que $R_2$ et $R_3$ représentent ensemble le radical cyclohexyle.

**4.** Procédé selon la revendication 1, caractérisés en ce que R représente le radical éthyle ou le radical isobutyle.

**5.** Procédé selon la revendication 1, caractérisé en ce que la réaction est effectuée par transfert de phase, en présence d'une base forte et d'un catalyseur tel que le chlorure de benzyltriéthylammonium.

**6.** Procédé selon la revendication 1, caractérisé en ce qu'on obtient les dérivés halogénés de formule I ou II en traitant une 2-($\alpha$-hydroxy-$\beta$-alkoxy) éthyl pyrrolidine ou-pipédidine par un agent d'halogénation.

**7.** Procédé selon la revendication 6, caractérisé en ce que l'halogénation est effectuée au moyen de chlorure de thionyl.

**Claims**

**Claims for the following Contracting States : AT, BE, CH, DE, FR, GB, IT, LI, NL, SE**

**1.** Compounds of formula:

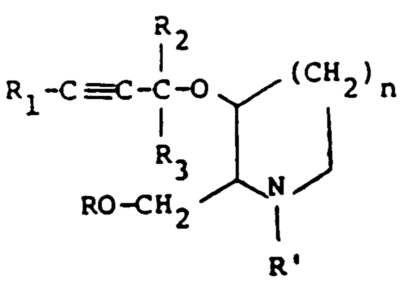

in which R and R' represent an alkyl radical having 1 to 7 carbon atoms; $R_1$ represents hydrogen or an alkyl radical having 1 to 7 carbon atoms; $R_2$ and $R_3$ represent separately an alkyl radical having 1 to 7 carbon atoms or the phenyl radical or together with the carbon atom to which they are attached, a cycloalkyl radical having not more than 7 carbon atoms; $\underline{n}$ may take the values 2 or 3, and their pharmaceutically acceptable salts.

**2.** Compounds according to Claim 1, characterised in that in that the substituents R', $R_1$, $R_2$ or $R_3$ represent the methyl radical.

**3.** Compounds according to Claim 1, characterised in that $R_2$ and $R_3$ together represent the cyclohexyl radical.

**4.** Compounds according to Claim 1, characterised in that R represents the ethyl radical or the isobutyl radical.

5. Process for preparing the compounds according to any one of Claims 1 to 4, characterised in that a halogenated derivative of formula I or II

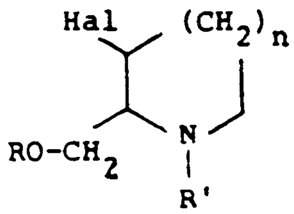

( I )

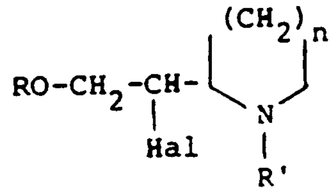

( II )

(in which R and R' have the meanings in Claim 1 and "Hal" designates a halogen) is reacted with an acetylenic alcohol of formula III

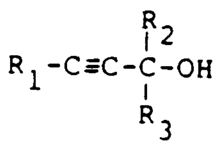

( III )

(in which $R_1$, $R_2$ and $R_3$ have the meanings in Claim 1)
optionally followed by conversion into a pharmaceutically acceptable salt.

6. Process according to Claim 5, characterised in that the reaction is carried out by phase transfer, in the presence of a strong base and a catalyst such as benzyltriethylammonium chloride.

7. Process according to Claim 5, characterised in that the halogenated derivatives of formula I or II are obtained by treating a 2-($\alpha$-hydroxyl-$\beta$-alkoxy)ethyl-pyrrolidine or -piperidine with a halogenating agent.

8. Process according to Claim 7, characterised in that the halogenation is carried out by means of thionyl chloride.

9. Pharmaceutical composition useful as a medicinal substance, characterised in that it contains as active ingredient at least one of the compounds according to Claims 1 to 4.

**Claims for the following Contracting States : ES, GR**

1. Process for preparing the compounds of formula:

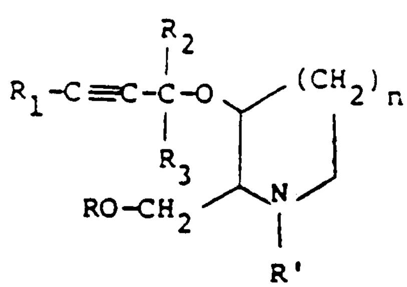

in which R and R' represent an alkyl radical having 1 to 7 carbon atoms; $R_1$ represents hydrogen or an alkyl radical having 1 to 7 carbon atoms; $R_2$ and $R_3$ represent separately an alkyl radical having 1 to 7 carbon atoms or the phenyl radical or together with the carbon atom to which they are attached, a cycloalkyl radical having not more than 7 carbon atoms; n may take the values 2 or 3, and their pharmaceutically acceptable salts, characterised in that a halogenated derivative of formula I or II

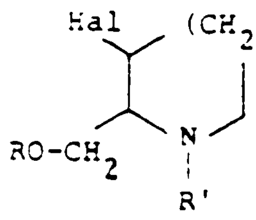

(I)

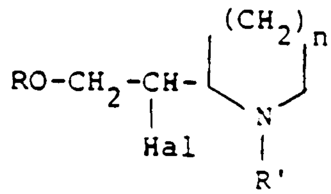

(II)

(in which R and R' have the meanings given above and "Hal" designates a halogen) is reacted with an acetylenic alcohol of formula III

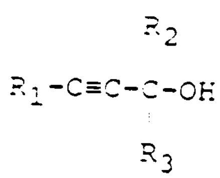

(III)

(in which $R_1$, $R_2$ and $R_3$ have the meanings given above)
optionally followed by conversion into a pharmaceutically acceptable salt.

**2.** Process according to Claim 1, characterised in that the substituents R', $R_1$, $R_2$ or $R_3$ represent the methyl radical.

**3.** Process according to Claim 1, characterised in that $R_2$ and $R_3$ together represent the cyclohexyl radical.

**4.** Process according to Claim 1, characterised in that R represents the ethyl radical or the isobutyl radical.

**5.** Process according to Claim 1, characterised in that the reaction is carried out by phase transfer, in the presence of a strong base and a catalyst such as benzyltriethylammonium chloride.

**6.** Process according to Claim 1, characterised in that the halogenated derivatives of formula I or II are obtained by treating a 2-($\alpha$-hydroxyl-$\beta$-alkoxy)ethyl-pyrrolidine or -piperidine with a halogenating agent.

**7.** Process according to Claim 6, characterised in that the halogenation is carried out by means of thionyl chloride.

**Patentansprüche**
**Patentansprüche für folgende Vertragsstaaten : AT, BE, CH, DE, FR, GB, IT, LI, NL, SE**

**1.** Verbindungen der Formel:

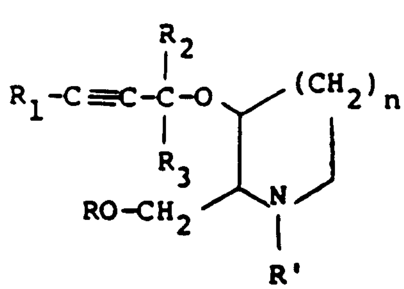

worin R und R' für einen Alkylrest mit 1 bis 7 Kohlenstoffatomen; $R_1$ für Wasserstoff oder einen

Alkylrest mit 1 bis 7 Kohlenstoffatomen; $R_2$ und $R_3$ getrennt für einen Alkylrest mit 1 bis 7 Kohlenstoffatomen oder für den Phenylrest oder zusammen mit dem Kohlenstoffatom, an das sie gebunden sind, für einen Cycloalkylrest mit höchstens 7 Kohlenstoffatomen stehen; wobei n die Werte 2 oder 3 annehmen kann, und ihre pharmazeutisch verträglichen Salze.

**2.** Verbindungen nach Anspruch 1, dadurch gekennzeichnet, daß die Substituenten R', $R_1$, $R_2$ oder $R_3$ für den Methylrest stehen.

**3.** Verbindungen nach Anspruch 1, dadurch gekennzeichnet, daß $R_2$ und $R_3$ zusammen für den Cyclohexylrest stehen.

**4.** Verbindungen nach Anspruch 1, dadurch gekennzeichnet, daß R für den Ethylrest oder den Isobutylrest steht.

**5.** Verfahren zur Herstellung der Verbindungen nach einem der Ansprüche 1 bis 4, dadurch gekennzeichnet, daß man ein halogeniertes Derivat der Formel I oder II

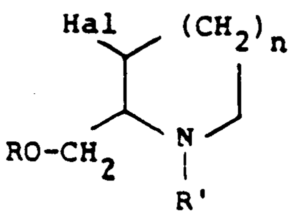

(I)

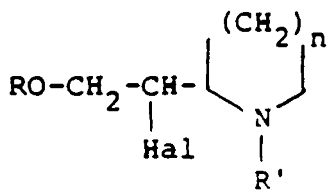

(II)

(worin R und R' die Bedeutungen des Anspruchs 1 aufweisen und "Hal" ein Halogen bezeichnet) mit einem acetylenischen Alkohol der Formel III

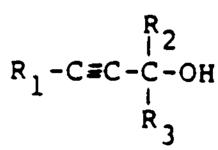

(III)

(worin $R_1$, $R_2$ und $R_3$ die Bedeutungen des Anspruchs 1 aufweisen) reagieren läßt und gegebenenfalls anschließend in ein pharmazeutisch verträgliches Salz umsetzt.

**6.** Verfahren nach Anspruch 5, dadurch gekennzeichnet, daß man die Umsetzung mittels Phasentransfer in Anwesenheit einer starken Base und eines Katalysators wie Benzyltriethylammoniumchlorid durchführt.

**7.** Verfahren nach Anspruch 5, dadurch gekennzeichnet, daß man die halogenierten Derivate der Formel I oder II erhält, indem man ein 2-($\alpha$-Hydroxy-$\beta$-alkoxy)ethyl-pyrrolidin oder -piperidin mit einem Halogenierungsmittel behandelt.

**8.** Verfahren nach Anspruch 7, dadurch gekennzeichnet, daß die Halogenierung mittels Thionylchlorid erfolgt.

**9.** Als Arzneimittel verwendbare pharmazeutische Zusammensetzung, dadurch geknnzeichnet, daß sie als wirkstoff mindestens eine der Verbindungen nach Ansprüchen 1 bis 4 enthält.

**Patentansprüche für folgende Vertragsstaaten : ES, GR**

**1.** Verfahren zur Herstellung von Verbindungen der Formel:

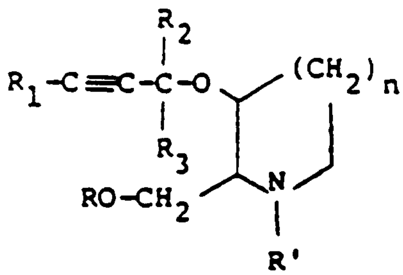

worin R und R' für einen Alkylrest mit 1 bis 7 Kohlenstoffatomen; $R_1$ für Wasserstoff oder einen Alkylrest mit 1 bis 7 Kohlenstoffatomen; $R_2$ und $R_3$ getrennt für einen Alkylrest mit 1 bis 7 Kohlenstoffatomen oder für den Phenylrest oder zusammen mit dem Kohlenstoffatom, an das sie gebunden sind, für einen Cycloalkylrest mit höchstens 7 Kohlenstoffatomen stehen; wobei n die Werte 2 oder 3 annehmen kann, und ihre pharmazeutisch verträglichen Salze, dadurch gekennzeichnet, daß man ein halogeniertes Derivat der Formel I oder II

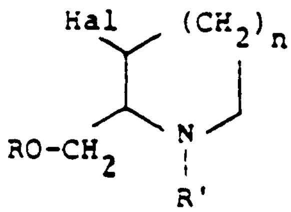

(I)

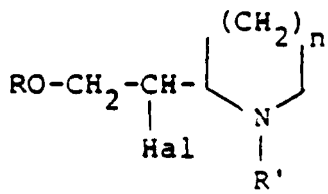

(II)

(worin R und R' die obengenannten Bedeutungen aufweisen und "Hal" ein Halogen bezeichnet) mit einem acetylenischen Alkohol der Formel III

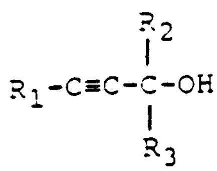

(worin $R_1$, $R_2$ und $R_3$ die obengenannten Bedeutungen aufweisen) reagieren läßt und gegebenenfalls anschließend in ein pharmazeutisch verträgliches Salz umsetzt.

2. Verfahren nach Anspruch 1, dadurch gekennzeichnet, daß die Substituenten R', $R_1$, $R_2$ oder $R_3$ für den Methylrest stehen.

3. Verfahren nach Anspruch 1, dadurch gekennzeichnet, daß $R_2$ und $R_3$ zusammen für den Cyclohexylrest stehen.

4. Verfahren nach Anspruch 1, dadurch gekennzeichnet, daß R für den Ethylrest oder den Isobutylrest steht.

5. Verfahren nach Anspruch 1, dadurch gekennzeichnet, daß man die Umsetzung mittels Phasentransfer in Anwesenheit einer starken Base und eines Katalysators wie Benzyltriethylammoniumchlorid durchführt.

6. Verfahren nach Anspruch 1, dadurch gekennzeichnet, daß man die halogenierten Derivate der Formel I oder II erhält, indem man ein 2-($\alpha$-Hydroxy-$\beta$-alkoxy)ethyl-pyrrolidin oder -piperidin mit einem Halogenierungsmittel behandelt.

7. Verfahren nach Anspruch 6, dadurch gekennzeichnet, daß die Halogenierung mittels Thionylchlorid erfolgt.